# EUROPÄISCHE PATENTSCHRIFT

(11) **EP 2 515 997 B1**
(45) Veröffentlichungstag und Bekanntmachung des Hinweises auf die Patenterteilung: **13.12.2017**
(21) Anmeldenummer: 10807698.5
(22) Anmeldetag: 22.12.2010
(51) Int. Cl.: A61N 1/04, A61N 1/40, A61N 1/44, H05H 1/24

(54) **Elektrodenanordnung für eine dielektrisch behinderte Plasmabehandlung und Verfahren zur Plasmabehandlung einer Oberfläche**
Electrode for a dielectric barrier discharge plasma treatment and a method for plasma treating a surface
Électrode pour le traitement par plasma par décharge à barrière diélectrique et un procédé pour le traitement par plasma d'une surface

(30) Priorität: 24.12.2009 DE 102009060627
(43) Veröffentlichungstag der Anmeldung: 31.10.2012
(73) Patentinhaber: Cinogy GmbH, 37115 Duderstadt (DE)
(72) Erfinder: WANDKE, Dirk, 37308 Heilbad Heiligenstadt (DE); SEGL, Maximilian, 37115 Duderstadt (DE); TRUTWIG, Leonhard, 37115 Duderstadt (DE)
(74) Vertreter: Lins, Edgar
(86) Internationale Anmeldenummer: PCT/DE2010/001513
(87) Internationale Veröffentlichungsnummer: WO 2011/076193

(56) Entgegenhaltungen:
- EP-A1- 1 933 605
- WO-A2-2007/067924
- DE-A1-102007 030 915
- US-A1- 2002 005 395

## Beschreibung

Die Erfindung betrifft eine Elektrodenanordnung für eine dielektrisch behinderte Plasmabehandlung einer als Gegenelektrode verwendeten unregelmäßig dreidimensional geformten Oberfläche eines elektrisch leitenden Körpers, mit einer flexiblen Elektrode und einem Dielektrikum, das zur Anordnung mit einem definierten Abstand zu der zu behandelnden Oberfläche zur Ausbildung eines kalten Plasmas ausgebildet ist wobei die Elektrodenanordnung aufgrund ihrer Flexibilität an die Kontur der Oberfläche anpassbar ist.

Die Erfindung betrifft ferner ein Verfahren zur Plasmabehandlung einer Oberfläche eines elektrisch leitenden Körpers unter Ausschluss eines menschlichen oder tierischen Körpers mit einer derartigen Elektrodenanordnung.

Dielektrisch behinderte Plasmaentladungen werden für zahlreiche Anwendungsfälle eingesetzt. Durch DE 195 32 105 C2 ist es bekannt, die Oberfläche von dreidimensionalen Werkstücken zu behandeln, beispielsweise zu aktivieren oder zu reinigen. Durch eine sogenannte Barriereentladung ist es möglich, eine Ölschicht bis auf minimale Ölbeläge zu reduzieren. Wesentlich ist dabei allerdings, dass eine gleichmäßige Behandlung der Oberfläche stattfindet. Hierfür ist eine homogene Ausbildung des Plasmas erforderlich, wobei die Vorstellung besteht, dass die Plasmaentladungen in voneinander beabstandeten dünnen Filamenten stattfinden. Problematisch ist dies bei unregelmäßig dreidimensional geformten Oberflächen. In der DE 195 32 105 C2 ist daher vorgesehen, von der Oberfläche des Werkstücks eine Negativform mit dem Dielektrikum auszubilden, das somit aus einem formbaren, beispielsweise pressbaren oder tiefziehbaren Kunststoff besteht. Vorgesehen ist dabei ferner, dass eine Zwischenschicht verwendet wird, sodass das Dielektrikum mit der Zwischenschicht unmittelbar an der Oberfläche des Werkstücks geformt werden kann. Die Zwischenschicht wird dann entfernt, um einen Zwischenraum zwischen dem Dielektrikum und der Elektrode zu gewährleisten, in dem sich das Plasma ausbilden kann. Das Dielektrikum wird auf seiner von der zu behandelnden Oberfläche abgewandten Seite mit einem leitenden Material beschichtet, dem die benötigte hohe Spannung in Form einer Wechselspannung zuführbar ist.

Durch DE 10 2007 030 915 A1 ist es bekannt, Hohlfasern aus einem dielektrischen Werkstoff auszubilden, die in ihrem Inneren mit einer metallisch leitenden Beschichtung versehen werden, sodass die Hohlfaser ein Dielektrikum mit einer inneren abgeschirmten Elektrode bildet, das zusammen mit der als Gegenelektrode dienenden Oberfläche eines leitenden Körpers ein Plasmafeld ausbilden kann. Dabei ist es auch vorgesehen, mit den Hohlfasern ein Gewebe auszubilden, das flächig auf eine unregelmäßige Oberfläche, insbesondere Hautoberfläche eines menschlichen Körpers auflegbar ist. Dadurch entsteht der Vorteil einer an die unregelmäßige Topologie der Hautoberfläche anpassbaren Elektrodenanordnung für die Durchführung einer Plasmabehandlung. Nachteilig hieran ist jedoch der hohe Fertigungsaufwand für die Ausbildung der das Gewebe bildenden Hohlfasern, die in ihrem Hohlraum eine flexible Elektrode aufweisen sollen, um die für die Anpassung an die Hautoberfläche erforderliche Flexibilität des Elektrodengewebes zu gewährleisten.

Der vorliegenden Erfindung liegt daher die Aufgabe zugrunde, eine Elektrodenanordnung zu schaffen, mit der eine sichere dielektrisch behinderte Plasmabehandlung der Oberfläche eines unregelmäßig dreidimensional geformten Körpers, insbesondere der Hautoberfläche eines Lebewesens, möglich ist und die einfacher und preiswerter herzustellen ist.

Diese Aufgabe wird erfindungsgemäß mit einer Elektrodenanordnung mit den Merkmalen des Patentanspruchs 1 gelöst.

Als Ausgangsmaterial für die erfindungsgemäße Elektrodenanordnung dient daher vorzugsweise ein ebenes flächiges Dielektrikum, das auf seiner von der zu behandelnden Oberfläche abgewandten Rückseite eine ebene flächige Elektrode trägt, sodass eine flexible Platte gebildet wird, die aufgrund ihrer Flexibilität an die Kontur der Oberfläche anpassbar ist, auch wenn diese dreidimensional geformt ist und insbesondere eine unregelmäßige dreidimensionale Topografie aufweist. Für die Erfindung ist es wesentlich, dass das Dielektrikum eine durchgehende Schicht bildet, mit der die Elektrode von der zu behandelnden Oberfläche abgeschirmt wird. Darüber hinaus bildet das Dielektrikum zur zu behandelnden Oberfläche hin eine Struktur aus, in der sich Bereiche befinden, in denen Luft geführt und ausgetauscht werden kann, um in diesen Bereichen das kalte Plasma entstehen zu lassen, mit dem die Oberfläche, insbesondere die Hautoberfläche eines menschlichen oder tierischen Körpers, behandelt werden kann. Bevorzugt ist eine kosmetische Behandlung der Oberfläche, durch die diese kosmetische Wirkstoffe verbessert aufnehmen kann, wodurch in effizienter Weise kosmetische Behandlungen, wie Faltenglättungen und Porenverkleinerungen möglich sind. Eine weitere wirksame Behandlung kann durch die Ausbildung der Elektrodenanordnung als Einlegesohle erreicht werden. Dabei entsteht eine bakterizide und fungizide Wirkung auf die Fußhaut, wobei das Dielektrikum direkt oder über einen Strumpf an der Fußhaut, insbesondere an der Fußsohle, anliegen kann.

Die flächige Elektrode ist vorzugsweise in das Dielektrikum eingebettet. Dies kann auf der Rückseite der Schicht des Dielektrikums erfolgen, die die Elektrode von der zu behandelnden Oberfläche abschirmt. Es ist aber auch möglich, die flächige Elektrode vollständig in das Dielektrikum einzubetten. In allen Fällen kann die flächige Elektrode mit einem aus dem Dielektrikum herausgeführten Anschluss kontaktierbar sein.
Die Elektrode ist vorzugsweise durch ein flexibles Drahtgitter gebildet, weil sich dieses für eine flexible Elektrode einfach und kostengünstig herstellen lässt.
Die Struktur des Dielektrikums ist einer bevorzugten Ausführungsform einstückig mit dem Rest der Schicht des Dielektrikums ausgebildet, die die Elektrode von der zu behandelnden Oberfläche abschirmt. Die Strukturierung definiert dabei die Tiefe der Bereiche, in denen sich Luft zur Ausbildung des Plasmas befinden kann, wenn das Dielektrikum unmittelbar auf die Oberfläche aufgelegt wird. Wenn das Dielektrikum aus einem geeigneten Kunststoff, beispielsweise thermoplastischem Polyethylen, gebildet ist, kann die gewünschte Struktur beim Gießen des flächigen Dielektrikums eingearbeitet werden.
Es ist aber auch möglich, die Struktur als separat ausgebildete Lage mit dem Rest der Schicht des Dielektrikums zu verbinden. In diesem Fall kann die Struktur auch feinfaserig gebildet sein, und beispielsweise ein Gewebe sein, wodurch die luftführenden Bereiche ggf. vergrößert werden und eine gleichmäßigere Ausbildung des Plasmas erreicht wird.
Bei einer fertigungstechnisch einfacheren Ausbildung der Struktur aus vorstehenden Noppen wird für luftdurchlässige Zwischenräume gesorgt, in denen sich das Plasma ausbilden kann. Die Noppen können dabei vorzugsweise kreiszylindrisch, kegelförmig oder kegelstumpfförmig ausgebildet sein. Ferner haben die Noppen vorzugsweise eine Höhe zwischen 0,5 und 10 mm, bevorzugt zwischen 1 und 8 mm und weiter bevorzugt zwischen 2 und 3 mm.
Die Erfindung soll im Folgenden anhand eines in den Zeichnungen dargestellten Ausführungsbeispiels näher erläutert werden. Es zeigen:
- Figur 1: die Teile einer erfindungsgemäßen Elektrodenanordnung in einer explodierten Darstellung;
- Figur 2: eine Draufsicht auf die Vorderseite der gemäß Figur 1 zusammengestellten Elektrodenanordnung;
- Figur 3: einen Längsschnitt durch die Elektrodenanordnung gemäß Figur 2;
- Figur 4: eine perspektivische Ansicht einer an eine dreidimensional geformte Oberfläche angepasste Elektrodenanordnung;
- Figur 5: einen Längsschnitt durch die Elektrodenanordnung entlang der Linie V-V in Figur 4.

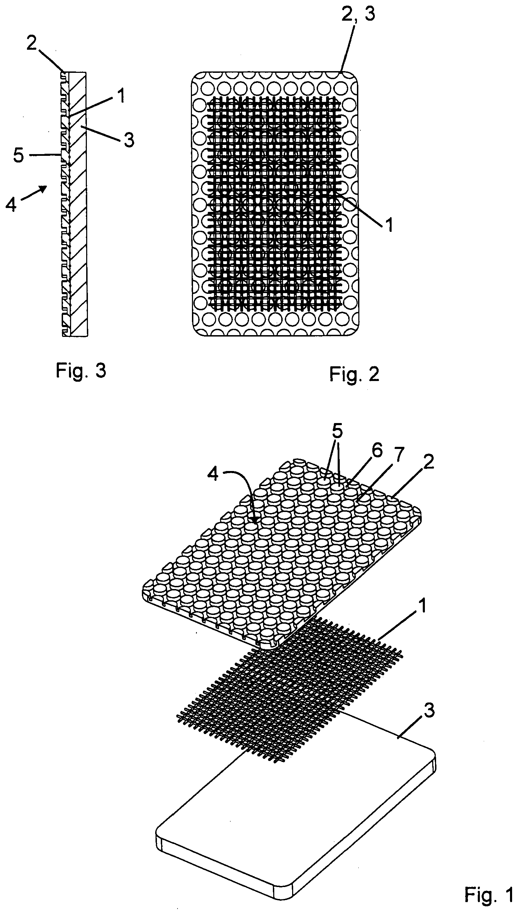

Figur 1 verdeutlicht, dass die erfindungsgemäße Elektrodenanordnung eine Elektrode 1 aufweist, die als im Ausgangszustand ebenes, flexibles Elektrodengitter aus Metall ausgebildet ist. Die Elektrode ist zwischen einer vorderen Schicht 2 aus einem dielektrischen Material und einer rückwärtigen Schicht 3 aus einem dielektrischen Material angeordnet. Die beiden dielektrischen Schichten 2, 3 sind im Ausgangszustand eben und flächig ausgebildet und überragen die Elektrode 1 an allen vier Seitenkanten, sodass die Elektrode 1 allseitig in das durch die beiden Schichten 2, 3 gebildete Dielektrikum eingebettet ist. Hierzu sind die Schichten 2, 3 miteinander verbunden, vorzugsweise flächig. Die Verbindung kann beispielsweise durch Verkleben oder Verschweißen erfolgen. Selbstverständlich können mit externen Verbindungsmitteln auch lösbare Verbindungen realisiert werden, jedoch mit einem höheren Aufwand. Die in das Dielektrikum eingebettete Elektrode 1 ist mit einem aus dem Dielektrikum herausragenden (nicht dargestellten) Anschluss kontaktierbar.
Die vordere Schicht 2 des Dielektrikums ist auf der von der Elektrode 1 abgewandten Seite mit einer strukturierten Oberfläche 4 versehen. In dem dargestellten Ausführungsbeispiel wird die strukturierte Oberfläche durch vorragende Noppen 5 gebildet, die einen Abstand 6 zueinander aufweisen, sodass die strukturierte Oberfläche 4 zahlreiche miteinander verbundene Luftführungsbereiche 7 aufweist, in denen Luft strömen kann, wenn die Elektrodenanordnung mit den Noppen 5 ihrer vorderen Schicht 2 an einer zu bearbeitenden Oberfläche, beispielsweise an der Haut eines Lebewesens, anliegt.
Figur 2 verdeutlicht, dass die Elektrode 1 in Draufsicht mit Abstand von den Seitenkanten der Schichten 2, 3 endet, wobei die Schichten 2, 3 vorzugsweise gleich groß ausgebildet sind.
Der Längsschnitt der Figur 3 verdeutlicht die Einbettung der Elektrode 1 zwischen den Schichten 2, 3 und die Noppen 5 der strukturierten Oberfläche 4 der vorderen Schicht 2, die die Elektrode 1 gegenüber der zu bearbeitenden Oberfläche abschirmt.

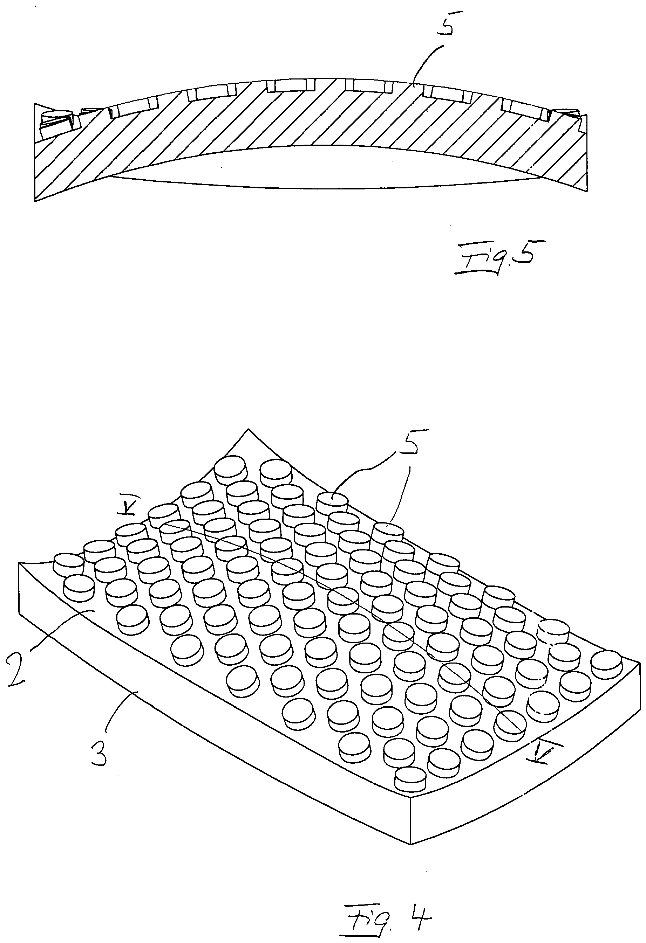

Figur 4 zeigt in einer perspektivischen Ansicht die Elektrodenanordnung gemäß Figur 1, wobei die beiden Schichten 2, 3 des Dielektrikums nicht mehr voneinander getrennt sind, weil sie beispielsweise durch einen flächigen Schweißvorgang miteinander verbunden sind. Das so gebildete Dielektrikum 2, 3 ist flexibel, weil die Schichten 2, 3 einerseits und die darin eingebettete Elektrode 1 andererseits flexibel sind. Demgemäß kann sich die Elektrodenanordnung an die Form einer dreidimensional geformten Oberfläche anpassen, wie dies in Figur 4 schematisch angedeutet ist. Die zu behandelnde Oberfläche ist dabei nicht dargestellt. Es ist erkennbar, dass eine von oben gesehen konvexe mittige Wölbung zu den diagonal gegenüberliegenden Enden in eine konkave Wölbung übergeht. Damit wird die Anpassbarkeit der Elektrodenanordnung auch an unregelmäßige Wölbungen der zu behandelnden Oberfläche verdeutlicht.
Der entlang der Linie V-V in Figur 4 dargestellte Längsschnitt der Figur 5 verdeutlicht die mittige konvexe Wölbung und lässt, insbesondere am linken Ende eine nach hinten ansteigende konkave Wölbung erkennen.

Es ist ohne weiteres ersichtlich, dass die erfindungsgemäße Elektrodenanordnung außerordentlich einfach herzustellen ist und in einfacher Weise eine Behandlung einer auch unregelmäßig gewölbten Oberfläche ermöglicht.

Die Figuren 4 und 5 zeigen die Verschmelzung der beiden dielektrischen Schichten 2, 3 zu einem einheitlichen Bauteil, in das die Elektrode 1 eingeschlossen ist.
Es ist ohne weiteres möglich, die strukturierte Oberfläche 4 auch als separates flächiges Element auszubilden, das mit dem Rest der die Elektrode 1 abschirmenden Schicht 2 verbunden wird. Beispielsweise kann die strukturierte Oberfläche in dieser Weise durch ein flächiges flexibles Textil- oder Kunststoffgewebe gebildet sein, das sich zwar als abschirmende Schicht 2 nicht eignet, jedoch als strukturierte Oberfläche 4 geeignet ist, Luftführungsbereiche auszubilden, in denen durch die angelegte hohe Wechselspannung das zur Behandlung benötigte Plasma ausgebildet wird.
Ebenso ist es für manche Anwendungsfälle denkbar, die Elektrode 1 lediglich auf die Rückseite der dielektrischen Schicht 2 aufzubringen. Im Allgemeinen wird es aber sinnvoll sein, die Elektrode 1 durch eine isolierende Schicht abzudecken, um unerwünschte Spannungsüberschläge, beispielsweise auf eine Bedienperson, auf der Rückseite der abschirmenden Schicht 2 zu vermeiden. Daher stellt die Einbettung der Elektrode 1 in das Dielektrikum 2, 3 eine bevorzugte Ausführungsform dar.
Das dargestellte Ausführungsbeispiel geht von im Ausgangszustand ebenen flächigen Schichten 2, 3 und einer ebenen Elektrode 1 aus. Für Anwendungen, die beispielsweise insbesondere für konvex gekrümmte Oberflächen vorgesehen sind, kann es auch sinnvoll sein, in konvexer Krümmung vorgeformte flächige Schichten 2, 3 und eine entsprechend vorgeformte flächige Elektrode 1 zu verwenden. Entsprechendes gilt für andere Vorformungen, die die Anpassung an die zu behandelnden Oberflächen erleichtert, wobei die Flexibilität der Elektrodenanordnung in jedem Fall gewahrt bleibt und eine präzise Anpassung an die Oberfläche ermöglicht.

## Patentansprüche

1. Elektrodenanordnung für eine dielektrisch behinderte Plasmabehandlung einer als Gegenelektrode verwendeten unregelmäßig dreidimensional geformten Oberfläche eines elektrisch leitenden Körpers, mit einer flexiblen Elektrode (1) und einem flexiblen Dielektrikum (2, 3), das zur Anordnung mit einem definierten Abstand zu der zu behandelnden Oberfläche zur Ausbildung eines kalten Plasmas ausgebildet ist, wobei die Elektrodenanordnung flexibel ausgebildet ist und eine Vorderseite und eine Rückseite aufweist und aufgrund ihrer Flexibilität an die Kontur der Oberfläche anpassbar ist, **dadurch gekennzeichnet, dass** das Dielektrikum (2, 3) durch ein flächiges, eine durchgehende Schicht bildendes Material gebildet ist, wobei die durchgehende Schicht auf seiner zur zu behandelnden Oberfläche zeigenden Seite mit einer Struktur (4) versehen ist, um Luftführungsbereiche (7) auszubilden, wenn das Dielektrikum (2, 3) auf der zu behandelnden Oberfläche aufliegt, und dass die Elektrode (1) am Dielektrikum (2, 3) so befestigt ist, dass die durchgehende Schicht (2) des Dielektrikums (2, 3) die Elektrode (1) von der zu behandelnden Oberfläche abschirmt.

2. Elektrodenanordnung nach Anspruch 1, **dadurch gekennzeichnet, dass** die Elektrode (1) in das Dielektrikum (2, 3) eingebettet und mit einem aus dem Dielektrikum (2, 3) herausgeführten Anschluss kontaktierbar ist.

3. Elektrodenanordnung nach Anspruch 2, **dadurch gekennzeichnet, dass** die Elektrode in das Dielektrikum (2, 3) allseitig eingebettet ist.

4. Elektrodenanordnung nach einem der Ansprüche 1 bis 3, **dadurch gekennzeichnet, dass** das Dielektrikum (2, 3) für eine Anlage auf der Haut eines Lebewesens ausgebildet ist.

5. Elektrodenanordnung nach einem der Ansprüche 1 bis 4, **dadurch gekennzeichnet, dass** die Elektrode (1) durch ein flexibles Drahtgitter aus Metall gebildet ist.

6. Elektrodenanordnung nach einem der Ansprüche 1 bis 5, **dadurch gekennzeichnet, dass** die Elektrode (1) aus Carbonfasern oder Carbonfasergeflecht gebildet ist.

7. Elektrodenanordnung nach einem der Ansprüche 1 bis 6, dadurch gekennzeicnet, dass die Struktur (4) des Dielektrikums (2, 3) einstückig in der Schicht (2) des Dielektrikums (2, 3) ausgebildet ist, die die Elektrode (1) von der zu behandelnden Oberfläche abschirmt.

8. Elektrodenanordnung nach einem der Ansprüche 1 bis 6, **dadurch gekennzeichnet, dass** die Struktur (4) als separat ausgebildete Lage mit dem Rest der Schicht (2) des Dielektrikums (2, 3) verbunden ist, die die Elektrode (1) von der zu behandelnden Oberfläche abschirmt.

9. Elektrodenanordnung nach einem der Ansprüche 1 bis 8, **dadurch gekennzeichnet, dass** die Struktur (4) aus vorstehenden Noppen (5) besteht, die luftdurchlässige Zwischenräume (6) ausbilden.

10. Elektrodenanordnung nach Anspruch 9, **dadurch gekennzeichnet, dass** die Noppen (5) kreiszylindrisch, kegelförmig oder kegelstumpfförmig ausgebildet sind.

11. Verfahren zur Plasmabehandlung einer Oberfläche eines elektrisch leitenden Körpers unter Ausschluss eines menschlichen oder tierischen Körpers mittels einer flexiblen Elektrodenanordnung, die auf die Oberfläche aufgelegt und an die Kontur der Oberfläche angepasst wird, **dadurch gekennzeichnet, dass** eine Elektrodenanordnung nach einem der Ansprüche 1 bis 10 verwendet wird, die mit der zur Oberfläche hin ausgebildeten Struktur (4) der durchgehenden Schicht des Dielektrikums (2, 3) auf die Oberfläche aufgelegt wird.

12. Verfahren nach Anspruch 11, **dadurch gekennzeichnet, dass** die Elektrodenanordnung auf eine unregelmäßig dreidimensional geformte Oberfläche aufgelegt wird.

## Claims

1. An electrode arrangement for a dielectric barrier discharge plasma treatment of an irregularly three-dimensionally shaped surface of an electrically conducting body, which surface is used as a counter electrode, comprising a flexible electrode (1) and a flexible dielectric (2, 3) which is designed to be arranged at a defined distance from the surface to be treated in order to form a cold plasma, wherein the electrode arrangement is designed flexible and provided with a front side and a rear side and is upon its flexibility adaptable to the contour of the surface, **characterized in that** the dielectric (2, 3) is formed by a planar material forming a continuous layer wherein the continuous layer is provided with a structure (4) on the side of said material facing the surface to be treated, in order to form air guiding areas (7) when the dielectric (2, 3) lies on the surface to be treated, and that the electrode (1) is fastened to the dielectric (2, 3) in such a way that the continuous layer (2) of the dielectric (2, 3) shields the electrode (1) from the surface to be treated.

2. The electrode arrangement as claimed in claim 1, **characterized in that** the electrode (1) is embedded in the dielectric (2, 3) and is designed to be brought into contact with a terminal which leads out of the dielectric (2, 3).

3. The electrode arrangement as claimed in claim 2, **characterized in that** the electrode is embedded in the dielectric (2, 3) on all sides.

4. The electrode arrangement as claimed in any one of claims 1 to 3, **characterized in that** the dielectric (2, 3) is designed to rest on the skin of a living organism.

5. The electrode arrangement as claimed in any one of claims 1 to 4, **characterized in that** the electrode (1) is formed by a flexible wire mesh made of metal.

6. The electrode arrangement as claimed in any one of claims 1 to 5, **characterized in that** the electrode (1) is formed of carbon fibers or carbon fiber mesh.

7. The electrode arrangement as claimed in any one of claims 1 to 6, **characterized in that** the structure (4) of the dielectric (2, 3) is formed integrally in the layer (2) of the dielectric (2, 3) which shields the electrode (1) from the surface to be treated.

8. The electrode arrangement as claimed in any one of claims 1 to 6, **characterized in that** the structure (4) as a separately constructed layer is connected with the rest to that of the layer (2) of the dielectric (2, 3) which shields the electrode (1) from the surface to be treated.

9. The electrode arrangement as claimed in any one of claims 1 to 8, **characterized in that** the structure (4) consists of projecting elevations (5), which form air-permeable intermediate spaces (6).

10. The electrode arrangement as claimed in claim 9, **characterized in that** the elevations (5) are implemented in a circular-cylindrical, conical or frustoconical form.

11. A method for plasma treatment of a surface of an electrically conducting body excluding a human or animal body by a flexible electrode arrangement which is placed on the surface and adapted to the contour of the surface, **characterized in that** an electrode arrangement as claimed in any one of claims 1 to 10 is used, which is formed with the structure (4) of the continuous layer of the dielectric (2, 3) in the direction of the surface and placed on the surface and adapted to the contour of the surface.

12. The method as claimed in claim 11, **characterized in that** the electrode arrangement is placed on an irregularly three-dimensionally shaped surface.

## Revendications

1. Agencement d'électrode pour le traitement d'une surface d'un corps électriquement conducteur par un plasma à barrière diélectrique, la surface étant utilisée à titre d'électrode antagoniste et étant de forme tridimensionnelle irrégulière, comportant une électrode flexible (1) et un diélectrique flexible (2, 3) qui est réalisé pour la disposition à une distance définie de la surface à traiter afin de réaliser un plasma froid, l'agencement d'électrode étant réalisé flexible et comprenant une face avant et une face arrière et pouvant être adapté au contour de la surface de par sa flexibilité, **caractérisé en ce que** le diélectrique (2, 3) est formé par un matériau surfacique constituant une couche continue, la couche continue étant pourvue d'une structure (4) sur son côté dirigé vers la surface à traiter, afin de réaliser des zones de guidage d'air (7) lorsque le diélectrique (2, 3) repose sur la surface à traiter, et **en ce que** l'électrode (1) est fixée sur le diélectrique (2, 3) de telle sorte que la couche continue (2) du diélectrique (2, 3) protège l'électrode (1) vis-à-vis de la surface à traiter.

2. Agencement d'électrode selon la revendication 1, **caractérisé en ce que** l'électrode (1) est noyée dans le diélectrique (2, 3) et peut être mise en contact avec un raccord dépassant hors du diélectrique (2, 3).

3. Agencement d'électrode selon la revendication 2, **caractérisé en ce que** l'électrode est noyée sur tous les côtés dans le diélectrique (2, 3).

4. Agencement d'électrode selon l'une des revendications 1 à 3, **caractérisé en ce que** le diélectrique (2, 3) est réalisé pour être mis en appui sur la peau d'un être vivant.

5. Agencement d'électrode selon l'une des revendications 1 à 4, **caractérisé en ce que** l'électrode (1) est réalisée par un treillis flexible de fils métalliques.

6. Agencement d'électrode selon l'une des revendications 1 à 5, **caractérisé en ce que** l'électrode (1) est constituée par des fibres de carbone ou par un tressage de fibres de carbone.

7. Agencement d'électrode selon l'une des revendications 1 à 6, **caractérisé en ce que** la structure (4) du diélectrique (2, 3) est réalisée d'un seul tenant dans la couche (2) du diélectrique (2, 3) qui protège l'électrode (1) vis-à-vis de la surface à traiter.

8. Agencement d'électrode selon l'une des revendications 1 à 6, **caractérisé en ce que** la structure (4) est reliée en tant que couche réalisée séparément au reste de la couche (2) du diélectrique (2, 3) qui protège l'électrode (1) vis-à-vis de la surface à traiter.

9. Agencement d'électrode selon l'une des revendications 1 à 8, **caractérisé en ce que** la structure (4) est constituée par des boutons (5) en saillie qui constituent des intervalles (6) perméables à l'air.

10. Agencement d'électrode selon la revendication 9, **caractérisé en ce que** les boutons (5) sont réalisés sous forme cylindrique circulaire, conique ou tronconique.

11. Procédé pour le traitement d'une surface d'un corps électriquement conducteur par un plasma avec exclusion d'un corps humain ou animal au moyen d'un agencement d'électrode flexible qui est posé sur la surface et adapté au contour de la surface, **caractérisé en ce que** on utilise un agencement d'électrode selon l'une des revendications 1 à 10 que l'on pose sur la surface, la structure (4) de la couche continue du diélectrique (2, 3) étant dirigée vers ladite surface.

12. Procédé selon la revendication 11, **caractérisé en ce que** on pose l'agencement d'électrode sur une surface de forme tridimensionnelle irrégulière.
